Europäisches Patentamt

European Patent Office

Office européen des brevets

(11) Veröffentlichungsnummer: **0 258 490 B1**

(12) **EUROPÄISCHE PATENTSCHRIFT**

(45) Veröffentlichungstag der Patentschrift: **20.03.91**

(51) Int. Cl.⁵: **C12M 3/00**

(21) Anmeldenummer: **86115824.4**

(22) Anmeldetag: **14.11.86**

(54) **Vorrichtung zur Durchführung zellbiologischer Versuche.**

(30) Priorität: 30.07.86 CH 3058/86
22.08.86 CH 3386/86

(43) Veröffentlichungstag der Anmeldung:
**09.03.88 Patentblatt 88/10**

(45) Bekanntmachung des Hinweises auf die
Patenterteilung:
**20.03.91 Patentblatt 91/12**

(84) Benannte Vertragsstaaten:
**CH DE FR GB LI**

(56) Entgegenhaltungen:
**CH-A- 580 961**

**PHARMACOLOGY & THERAPEUTIKS, Band
21, 1983, Seiten 35-51, Pergamon Press Ltd,
GB; R. LANGER: "Implantable controlled release systems"**

(73) Patentinhaber: **CONTRAVES AG
Schaffhauserstrasse 580
CH-8052 Zürich(CH)**

(72) Erfinder: **Cogoli, Augusto, Dr.
Herzogstrasse 22
CH-8044 Zürich(CH)**
Erfinder: **Gruenblatt-Nordau, Claudy-Gabrielle
24, rue Chaptal
F-75009 Paris(FR)**
Erfinder: **Huber, Beat
Oetwilerstrasse 49
CH-8634 Hombrechtikon(CH)**
Erfinder: **Suter, Robert N.
Leimgrübelstrasse 16
CH-8052 Zürich(CH)**
Erfinder: **Tschopp, Alexander
Scheuchzerstrasse 67
CH-8006 Zürich(CH)**

## Beschreibung

Die Erfindung betrifft eine Vorrichtung zur Durchführung zellbiologischer Versuche, bei welcher in einem Gehäuse mindestens zwei für den Zu- und Abfluss eines Zellkulturmediums in Verbindung stehende Kammern vorgesehen sind.

Die Technik der Zellzüchtung ausserhalb von Organismen ist in wissenschaftlichen Laboratorien sowie in der Industrie eine an sich bekannte Methode. Hierbei werden pflanzliche, tierische oder menschliche Zellen in speziell ausgebildeten und mit Reaktionskammern versehenen Vorrichtungen oder Gefässen in einem Nährmedium zur Vermehrung gebracht, wobei nach einem Zellzyklus das Nährmedium normalerweise verbraucht und somit durch frisches Medium zu ersetzen ist.

Der Erfindung liegt die Aufgabe zugrunde, eine Vorrichtung mit einfachem Aufbau der genannten Art zu schaffen, die eine kontinuierliche Zufuhr des Mediums zu der Reaktionskammer gewährleistet, wobei die Vorrichtung auch im schwerelosen Raum oder bei unterschiedlicher Ausrichtung zur Schwerkraftrichtung verwendbar sein soll, ohne dass eine wesentliche Mithilfe von Bedienungspersonal erforderlich ist.

Diese Aufgabe wird nach der Erfindung dadurch gelöst, dass die in einem ersten Gehäuseteil angeordnete erste Kammer zur Aufnahme eines Betriebsmediums sowie einer osmotischen Flüssigkeitspumpe und die in einem zweiten Gehäuseteil angeordnete zweite Kammer zur Aufnahme des Zellkulturmediums ausgebildet ist, wobei zur kontinuierlichen Abgabe des Zellkulturmediums die zweite Kammer über mehrere, ein geschlossenes System bildende Kanäle mit der Flüssigkeitspumpe und der ersten Kammer in Verbindung steht.

Die erfindungsgemässe Vorrichtung bildet ein in sich geschlossenes System und gewährleistet einen automatischen Mediumaustausch ohne weitere Hilfsmittel. Die darin verwendete und an sich bekannte osmotische Flüssigkeitspumpe wurde ursprünglich für andere Zwecke entwickelt und ist im Handel mit verschiedenen Spezifikationen erhältlich.

Der Anwendungsbereich der erfindungsgemässen Vorrichtung ist nicht nur auf ein Weltraumlabor beschränkt sondern auch bei Studien in der Entwicklungsbiologie und in der Embryologie verwendbar, bei welchen Studien das Problem der kontinuierlichen Zugabe von Medien und anderen flüssigen Stoffen ebenfalls auftritt.

Bei allen zellbiologischen Versuchen ist es wichtig, dass unter absolut sterilen Bedingungen gearbeitet werden kann. Aus diesem Grunde wurden für die vorliegende Vorrichtung nur Materialien verwendet, die sich hitzesterilisieren (autoklavieren) lassen.

Da Zellkulturen im allgemeinen druckempfindlich sind, sollten sich in einem derartigen geschlossenen System keine Ueberdrücke aufbauen. Dies ist bei der vorliegenden Vorrichtung ebenfalls gewährleistet.

Weitere Merkmale der Erfindung ergeben sich aus der folgenden Beschreibung in Verbindung mit der Zeichnung und den Patentansprüchen.

Ausführungsbeispiele des Erfindungsgegenstandes werden nachstehend anhand der Zeichnungen erläutert. Es zeigt:

Fig. 1 in schematischer Darstellung eine erste Ausführungsform einer erfindungsgemässen Vorrichtung,

Fig. 2 im Querschnitt eine schematische Darstellung einer osmotischen Pumpe,

Fig. 3 in Schnittansicht eine zweite, bevorzugte Ausführungsform der Vorrichtung,

Fig. 4 eine in Schnittansicht und in demontiertem Zustand dargestellte dritte Ausführungsform der Vorrichtung, und

Fig. 5 einen Längsschnitt durch die Vorrichtung, gemäss Fig. 4.

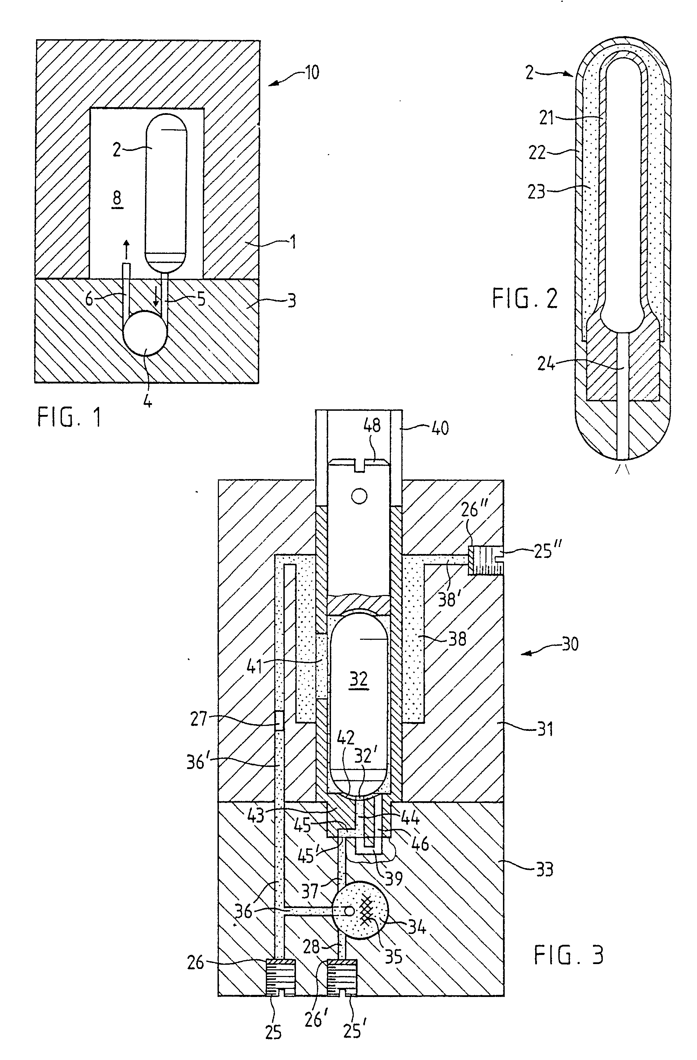

Eine erste, schematisch dargestellte Ausführung einer Vorrichtung 10 gemäss Fig. 1 zeigt eine von einem Gehäuse 1, 3 umschlossene zweite Kammer 8, die über einen Kanal 5 für den Zufluss und einen Kanal 6 für den Abfluss mit einer ersten Kammer 4, beispielsweise einer Reaktionskammer 4 verbunden ist. Bei dieser Vorrichtung 10 beginnt die Zufuhr eines Zellkulturmediums in die Reaktionskammer 4 sofort nach dem Einsetzen der osmotischen Pumpe 2 in die zweite Kammer 8 und Füllung derselben mit einem in die osmotische Pumpe 2 eindringenden Betriebsmedium. Das in der Reaktionskammer 4 verbrauchte Zellkulturmedium strömt über den Kanal 6 in die zweite Kammer 8 ab und kann sich dort mit dem Betriebsmedium für die osmotische Pumpe mischen. Es versteht sich, dass das Gehäuse 1, 3 der Vorrichtung 10 zum Einsetzen der Pumpe 2 und zum Füllen der beiden Kammern 8 und 4 geeignete Verschlussöffnungen oder Teilungsebenen aufweist, deren Ausführung dem jeweiligen Anwendungsbereich der Vorrichtung angepasst ist.

Die an sich bekannte osmotische Pumpe 2 hat einen inneren Speicherbehälter 21 mit undurchlässiger, flexibler Wand, die durch den osmotischen Druck in dem umgebenden semipermeablen Aussenbehälter 22 zusammengedrückt wird, so dass das zu fördernde Zellkulturmedium entsprechend durch einen Pumpenkanal 24 abströmt. In dem Raum 23 zwischen dem Aussenbehälter 22 und dem Speicherbehälter 21 befindet sich hierfür eine osmotische Substanz, die mit dem durch die semipermeable Wand eindringenden Betriebsmedium grössere Moleküle bildet, so dass diese durch diese Wand nicht nach aussen dringen können und sich der osmotische Druck in dem Raum 23 entwickelt, wodurch das Zellkulturmedium in gleichem Masse aus dem Speicherbehälter 21 verdrängt wird. In Fig. 1 und 3 ist eine derartige osmotische Pumpe 2, 32 in die erfindungsgemässe Vorrichtung 10 oder 30 eingesetzt dargestellt.

Die Vorrichtung 30 nach Fig. 3 ermöglicht im Unterschied zu derjenigen nach Fig. 1 den Start der Reaktion, d.h. die Zufuhr des Zellkulturmediums aus der Pumpe 32 zu der Reaktionskammer 34, durch eine einfache Drehbewegung einer Steuerhülse 40, nachdem die gefüllte Pumpe 32 und das Betriebsmedium bereits in die Vorrichtung 30 eingebracht worden sind. Hierfür ist die Pumpe 32 in die Steuerhülse 40 fest eingesetzt, so dass sie zusammen mit dieser relativ zu dem Gehäuse 31, 33 der Vorrichtung verdrehbar ist. Ein in die Steuerhülse 40 eingesetzter Verschlusszapfen 48 drückt den eine Austrittsöffnung 32' aufweisenden Bereich der Pumpe 32 gegen eine abdichtende Pumpenfassung 42 am Bodenteil 43 der Steuerhülse 40. Ein den Bodenteil 43 durchdringender Abströmkanal 44 mündet an seinem äusseren Bereich in eine Abzweigung 45, so dass eine Abströmöffnung 45' im Bodenteil 43 durch Drehen der Steuerhülse 40, um z.B. 90°, wahlweise zu einem Kanal 37 der Reaktionskammer 34 oder zu einem Bypass-Kanal 39 schwenkbar ist, über welchen Bypass-Kanal 39 das Zellkulturmedium unter Umgehung der Reaktionskammer 34 durch eine Oeffnung 46 im wesentlichen direkt in eine Kammer 38 strömt, die an die Pumpe 32 über mindestens eine Oeffnung 41 in der Steuerhülse 40 angrenzt und das Betriebsmedium enthält. In der Darstellung ist der Querschnitt des Bypass-Kanals 39 um 90° versetzt, so dass diese beide Stellpositionen der Steuerhülse 40 veranschaulicht. Ein Kanal 36, 36' für die Ableitung aus der Reaktionskammer 34, welcher zu der das Betriebsmedium einschliessenden und die Steuerhülse 40 mindestens teilweise umschliessenden Kammer 38 führt, weist vorzugsweise einen verhältnismässig kleinen Durchmesser auf, oder aber in dem Kanalstück 36' ist ein die Medien trennbar, schwimmender Kolben 27 angeordnet, so dass eine Durchmischung zwischen dem aus der Reaktionskammer abströmenden verbrauchten Zellkulturmedium und dem Betriebsmedium auf einen begrenzten Bereich beschränkt bleibt. Dies ermöglicht eine Untersuchung des verbrauchten Zellkulturmediums durch Absaugen mittels einer Injektionsspritze, entweder nach Oeffnen der Reaktionskammer 34 durch diese hindurch oder nach Oeffnen eines nich dargestellten Verschlusszapfens.

Zum Füllen der Reaktionskammer 34 mündet in diese weiterhin ein durch einen Zapfen 25' verschlossener Füllkanal 28, der auch für andere Zwe

ke, nach Entfernen des Verschlusszapfens 25', den Zugang, z.B. nach dem Durchstossen einer eingesetzten Gummi-Membrane 26' mittels einer nicht dargestellten Injektionsspritze, zu der Reaktionskammer 34 ermöglicht. Ein weiterer Verschlusszapfen 25″ mit Gummi-Membrane 26″ ermöglicht über einen Kanal 38' eine Entlüftung der Kammer 38. Die vor dem Verschlusszapfen 25, 25', 25″ angeordnete und in die entsprechend vorgesehene Oeffnung eingesetzte Gummi-Membrane 26, 26', 26″ (Dichtung) bewirkt nach dem Abziehen (Entfernen) der Injektionsspritze ein selbsttätiges Verschliessen, so dass keine Luft in das System gelangen kann. Für die Zurückhaltung von Feststoffpartikeln ist vor der Einmündung in die am unteren Ende durch den Verschlusszapfen 25 mit Gummi-Membrane 26 verschlossene Ableitung 36 in der Reaktionskammer 34 ein durch eine Schraffur schematisch angedeutetes Filterelement 35 eingesetzt. Für eine Beobachtung des Inhalts der Reaktionskammer 34, kann diese an ihren Enden durch nicht dargestellte Glasscheiben verschlossen sein. Mit Ausnahme der osmotischen Pumpe bestehen die Teile der Vorrichtung aus einem auf Sterilisationstemperatur erhitzbaren Material.

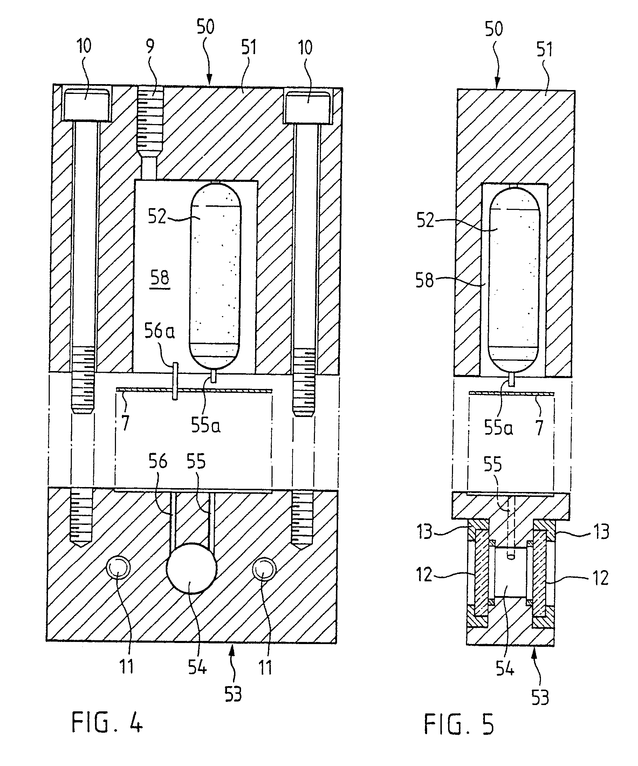

Die in Fig. 4 und 5 dargestellte Ausführungsform einer mit 50 bezeichneten Vorrichtung besteht im wesentlichen aus zwei funktionellen Hauptteilen; einem mit 51 bezeichneten ersten Gehäuseteil mit eingesetzter, osmotischer Flüssigkeitspumpe 52 und einem mit einer Zellkulturkammer 54 versehenen zweiten Gehäuseteil 53. Die Zellkulturkammer 54 besitzt einen als Eingang ausgebildeten ersten Kanal 55 und einen als Ausgang ausgebildeten zweiten Kanal 56, wobei in zusammengebautem Zustand am Kanal 55 eine Ausflussöffnung 55a der osmotischen Flüssigkeitspumpe 52 und am Kanal 56 eine Rückflusskanüle 56a angeschlossen sind. Die Rückflusskanüle 56a kann gegebenenfalls mit einem nicht dargestellten Einwegventil versehen werden. Um die Ausflussöffnung 55a gegen die Rückflusskanüle 56a gut abzudichten, werden beide durch eine Silikonmembrane 7 gestochen, die abdichtend nach Verschraubung des Gehäuseteils 51 mit dem Gehäuseteil 53 durch Schrauben 10 in einer entsprechenden Ausnehmung fixiert wird.

Das Füllen der Zellkulturkammer 54 wird folgendermassen bewerkstelligt: Nachdem durch ein Schauglashalter 13 ein erstes Schauglas 12 mit Hilfe von Schrauben 11 befestigt worden ist, kann die Zellkultur in die Zellkulturkammer 54 eingefüllt werden. Nach dem Einfüllen wird die im wesentlichen quer zu den Kanälen 55, 56 orientierte Zellkulturkammer 54 mit einem zweiten Schauglas 12 und einem zweiten Schauglashalter 13 mittels der Schrauben 11 dicht verschlossen. In dem Gehäuseteil 51 ist ein mit einem die Flüssigkeitspumpe 52 aufnehmenden Hohlraum 58 in Verbindung stehender Kanal 9 (Oeffnung) vorgesehen, welcher durch eine in Fig. 4 nicht dargestellte Schraube oder Stopfen verschlossen werden kann. In den Hohlraum 58 wird eine die osmotische Flüssigkeitspumpe 52 antreibende Flüssigkeit eingefüllt, welche das System in Betriebszustand versetzt. Die vorgängig durch die Ausflussöffnung 55a in die osmotische Flüssigkeitspumpe 52 eingefüllte Flüssigkeit (Medien etc.) wird kontinuierlich in die Zellkulturkammer 54 gepumpt. Das Abfliessen der verbrauchten Flüssigkeit durch die Rückflusskanüle 56a in den Hohlraum 58 verhindert das Auftreten eines Ueberdrucks in dem System

Der Zusammenbau der in Fig. 4 und Fig. 5 in demontiertem Zustand dargestellten Vorrichtung erfolgt im wesentlichen durch die strichpunktierten Linien, wobei die beiden Gehäuseteile 51 und 53 durch die Schrauben 10 gegeneinander gepresst werden und somit eine Baueinheit bilden.

## Ansprüche

1. Vorrichtung (10, 30, 50) zur Durchführung zellbiologischer Versuche, bei welcher in einem Gehäuse mindestens zwei für den Zu- und Abfluss eines Zellkulturmediums miteinander in Verbindung stehende Kammern vorgesehen sind, dadurch gekennzeichnet, dass die in einem ersten Gehäuseteil (1; 31; 51) angeordnete erste Kammer (8; 38; 58) ein Betriebsmedium sowie eine osmotische Flüssigkeitspumpe (2; 32; 52) enthält und die in einem zweiten Gehäuseteil (3; 33; 53) angeordnete zweite Kammer (4; 34; 54) das Zellkulturmedium enthält, wobei zur kontinuierlichen Abgabe des Zellkulturmediums die zweite Kammer (4; 34; 54) über mehrere, ein geschlossenes System bildende Kanäle mit der Flüssigkeitspumpe (2; 32; 52) und der ersten Kammer (8; 38; 58) in Verbindung steht.

2. Vorrichtung nach Anspruch 1, dadurch gekennzeichnet, dass die osmotische Pumpe (32) in eine um ihre Längsachse im Gehäuse (31) der Vorrichtung (30) drehbar gehaltene Steuerhülse (40) eingesetzt ist, die mindestens eine Oeffnung (41) aufweist, die die Verbindung von der das Betriebsmedium einschliessenden Kammer (38) zu der osmotischen Pumpe (32) herstellt, wobei im Bodenteil (43) der Steuerhülse (40) sich ein abgewinkelter Abströmkanal (44, 45) befindet, der durch Verdrehen der Steuerhülse zu dem Kanal (37) der Reaktionskammer (34) oder zu einem Bypass-Kanal (39) wahlweise ausrichtbar ist.

3. Vorrichtung nach Anspruch 1, dadurch gekennzeichnet, dass zwischen der Reaktionskammer (34) und der zweiten Kammer (38) für das Betriebsmedium der osmotischen Pumpe (32) ein langgestreckter Verbindungskanal (36, 36') vorgesehen ist, der die Durchmischung der zwischen den in beiden Kammern (34, 38) eingeschlossenen Medien begrenzt.

4. Vorrichtung nach Anspruch 3, dadurch gekennzeichnet, dass im oberen Teilstück des Gehäuseteils (31) in dem Verbindungskanal (36') ein die Medien trennender, frei schwimmender Kolben (27) angeordnet ist.

5. Vorrichtung nach Anspruch 3 und 4, dadurch gekennzeichnet, dass der Verbindungskanal (36, 36') über die Reaktionskammer (34) oder durch Entfernen eines Verschlusses (25, 25') für eine Probeentnahme zugänglich ist.

6. Vorrichtung nach Anspruch 1, dadurch gekennzeichnet, dass die zweite Kammer (54) der Vorrichtung (50) als eine das zweite Gehäuseteil (53) durchdringende, quer zu den Kanälen (55, 56) orientierte Ausnehmung ausgebildet und mittels zwei von Haltern (13) am Gehäuseteil (53) lösbar befestigte Schaugläser (12) flüssigkeitsdicht verschlossen ist.

7. Vorrichtung nach Anspruch 1, dadurch gekennzeichnet, dass die erste Kammer (38; 58) über einen das erste Gehäuseteil (31; 51) durchdringenden, verschliessbaren Kanal (38', 9) zugänglich ist.

8. Vorrichtung nach Anspruch 1, dadurch gekennzeichnet, dass mit Ausnahme der osmotischen Flüssigkeitspumpe (2; 32; 52) alle Teile aus hitzesterilisierbarem Material bestehen.

9. Vorrichtung nach Anspruch 1, dadurch gekennzeichnet, dass vor der Austrittsöffnung der Reaktionskammer (4; 34) ein Filterelement (35) angeordnet ist.

10. Vorrichtung nach Anspruch 1, dadurch gekennzeichnet, dass die zweite, als Reaktionskammer ausgebildete Kammer (4; 34) der Vorrichtung (10, 30) durch mindestens eine Glasscheibe verschlossen ist.

## Claims

1. Apparatus (10, 30, 50) for carrying out cytological tests, wherein at least two interconnected chambers for the in- and outflow of a cell culture medium are provided in a housing, characterised in that the first chamber (8; 38; 58) disposed in a first housing part (1; 31; 51) contains an operating medium and an osmotic fluid pump (2; 32; 52) and the second chamber (4; 34; 54) disposed in a second housing part (3; 33; 53) contains the cell culture medium, wherein, for continuous delivery of the cell culture medium, the second chamber (4; 34; 54) is connected to the fluid pump (2; 32; 52) and the first chamber (8; 38; 58) by way of several ducts which form a closed system.

2. Apparatus according to claim 1, characterised in that the osmotic pump (32) is inserted in a control sleeve (40) which is held in the housing (31) of the apparatus (30) capable of rotating about its longitudinal axis, which control sleeve has at least one opening (41) establishing the connection of the chamber (38) enclosing the operating medium with the osmotic pump (32), wherein there is located in the base part (43) of the control sleeve (40) an angled outflow duct (44, 45) which can be aligned as desired with the duct (37) of the reaction chamber (34) or with a bypass duct (39) by rotation of the control sleeve.

3. Apparatus according to claim 1, characterised in that an extended connecting duct (36, 36') is provided between the reaction chamber (34) and the second chamber (38) for the operating medium of the osmotic pump (32), said duct limiting intermixing between the media enclosed in the two chambers (34, 38).

4. Apparatus according to claim 3, characterised in that a freely floating piston (27) separating the media is disposed in the upper partial section of the housing part (31) in the connection duct (36').

5. Apparatus according to claim 3 and 4, characterised in that the connection duct (36, 36') is accessible for the removal of a sample via the reaction chamber (34) or by the removal of a seal (25, 25').

6. Apparatus according to claim 1, characterised in that the second chamber (54) of the apparatus (50) is designed as a recess passing through the second housing part (53) and aligned transversely to the ducts (55, 56) and is sealed fluid-tight by means of two observation glasses (12) detachably secured to the housing part (53) by holders (13).

7. Apparatus according to claim 1, characterised in that the first chamber (38; 58) is accessible by way of a closable duct (38', 9) which passes through the first housing part (31; 51).

8. Apparatus according to claim 1, characterised in that all the parts, with the exception of the osmotic fluid pump (2; 32; 52), are made of heat-sterilizable material.

9. Apparatus according to claim 1, characterised in that a filter element (35) is arranged in front of the outlet opening of the reaction chamber (4; 34).

10. Apparatus according to claim 1, characterised in that the second chamber (4; 34), designed as the reaction chamber, of the apparatus (10, 30) is closed by at least one glass pane.

**Revendications**

1. Dispositif (10, 30, 50) pour la réalisation d'expériences cytologiques comportant dans un boîtier, au moins deux chambres communiquant pour l'alimentation et l'évacuation d'un fluide de culture cytologique, dispositif caractérisé en ce que la première chambre (8, 38, 58) prévue dans une première partie de boîtier (1, 31, 51) reçoit un fluide fonctionnel ainsi qu'une pompe à liquide osmotique (2, 32, 52) et la seconde chambre (4, 34, 54) prévue dans la seconde partie de boîtier (3, 33, 53) contient le milieu de culture cytologique, et pour fournir en continu le milieu de culture cytologique, la seconde chambre (4, 34, 54) communique par plusieurs canaux formant un système fermé avec la pompe à liquide (2, 32, 52) et la première chambre (8, 38, 58).

2. Dispositif selon la revendication 1, caractérisé en ce que la pompe osmotique (32) est logée dans une douille de commande (40) maintenue libre en rotation autour de son axe longitudinal dans le boîtier (31) du dispositif (30), cette douille ayant au moins une ouverture (41) qui réalise la liaison de la chambre (38) entourant le fluide fonctionnel et la pompe osmotique (32), le fond (43) de la douille de commande (40) comportant un canal d'évacuation (44, 45) coudé qui, par rotation de la douille de commande, peut être aligné sélectivement sur le canal (37) de la chambre de réaction (34) ou sur un canal de dérivation (39).

3. Dispositif selon la revendication 1, caractérisé en ce qu'entre la chambre de réaction (34) et la seconde chambre (38) pour le fluide de fonctionnement de la pompe osmotique (32), il est prévu un canal de liaison (36, 36') allongé qui limite le mélange des milieux emprisonnés dans les deux chambres (34, 38).

4. Dispositif selon la revendication 3, caractérisé en ce que dans la pièce supérieure de la partie de boîtier (31) dans le canal de liaison (36') il est prévu un piston (27) flottant librement et qui sépare les milieux.

5. Dispositif selon la revendication 3 ou 4, caractérisé en ce que le canal de liaison (36, 36') est accessible pour une prise d'échantillon par la chambre (34) ou par enlèvement d'un bouchon (25, 25').

6. Dispositif selon la revendication 1, caractérisé en ce que la seconde chambre (54) du dispositif (50) est réalisée sous la forme d'une cavité traversant la seconde partie de boîtier (53), orientée transversalement aux canaux (55, 56) et elle fermée de manière étanche au liquide par deux vitres (12) fixées de manière amovible par des châssis (13) sur la partie de boîtier (53).

7. Dispositif selon la revendication 1, caractérisé en ce que la première chambre (38, 58) est accessible par un canal (38', 9) obturable, qui traverse la première partie de boîtier (31, 51).

8. Dispositif selon la revendication 1, caractérisé en ce qu'à l'exception de la pompe à liquide osmotique (2, 32, 52), toutes les pièces sont réalisées en un matériau stérilisable par élévation de température.

9. Dispositif selon la revendication 1, caractérisé par un élément de filtre (35) prévu en amont de l'orifice de sortie de la chambre de réaction (4, 34).

10. Dispositif selon la revendication 1, caractérisé en ce que la seconde chambre (4, 34) du dispositif (10, 30), réalisée sous forme de chambre à réaction, est fermée par au moins une vitre.

FIG. 1

FIG. 2

FIG. 3

FIG. 4

FIG. 5